# EUROPEAN PATENT SPECIFICATION

(11) **EP 2 844 320 B1**
(45) Date of publication and mention of the grant of the patent: **11.08.2021**
(21) Application number: 13720410.3
(22) Date of filing: 03.05.2013
(51) Int. Cl.: A61M 5/32

(54) **SHEATH PROTECTING A CANNULA, AND SAFETY SYRINGE COMPRISING SAID SHEATH**
SCHUTZHÜLLE FÜR EINE KANÜLE UND SICHERHEITSSPRITZE MIT DIESER HÜLLE
GAINE PROTÉGEANT UNE CANULE, ET SERINGUE DE SÉCURITÉ COMPRENANT LADITE GAINE

(30) Priority: 03.05.2012 WO PCT/EP2012/058160
(43) Date of publication of application: 11.03.2015
(73) Proprietor: SOFIC (Sté Française d'Instruments de Chirurgie), 81200 Mazamet (FR)
(72) Inventor: WOLLBOLD, Jürgen, F-81290 Labruguière (FR); COMBES, Christophe, F-81200 Mazamet (FR); LAMBERT, Grégory, F-92290 Châtenay-Malabry (FR); HAAS, Jérôme, F-91230 Montgeron (FR); ARNAUD, Cécile, F-75011 Paris (FR)
(74) Representative: Icosa
(86) International application number: PCT/EP2013/059310
(87) International publication number: WO 2013/164475

(56) References cited:
- EP-A1- 2 324 875
- FR-A1- 2 852 250
- US-A1- 2011 082 428
- US-A1- 2011 319 832
- US-B1- 6 669 671
- US-B1- 8 128 594
- None

## Description

### FIELD OF INVENTION

The present invention relates to a sheath protecting a cannula, said sheath being primarily designed to prevent or minimize accidental needlestick injuries. The present invention also relates to a safety syringe comprising said sheath, and more particularly to a safety syringe adapted to receive a medication-containing cartridge and comprising said sheath.

### BACKGROUND OF INVENTION

Needlestick injuries frequently occur among healthcare workers, introducing high risk of blood-borne pathogen infection for surgeons, assistants, and nurses, such as HIV, hepatitis B, hepatitis C or viral hemorrhagic fevers. In a UK report, 37% of nurses reported that they have sustained a needle-stick injury at some stage during their career (Prevention CfDCa, Overview: Risks and Prevention of Sharps Injuries in Healthcare Personnel, CDC, Atlanta, Ga, USA, 2004). These results have further to be read taking into account that not all needle-stick injuries are reported, and that the rate of detection may be low. In a study investigating the use of blunt needles during obstetrical laceration repair surgeries (Wilson et al., "The use of blunt needles does not reduce glove perforations during obstetrical laceration repair", Am J Obstet Gynecol., 2008, 199(6):641.el-3), only 11% of glove perforations were detected by the physician in a study.

Strategies are available in response to these issues, including education of healthcare workers on the risks and precautions, reduction of invasive procedures, management of exposures and use of safer devices.

Among the safest devices, an efficient way to protect a healthcare worker from being pricked by a needle is the use of safety syringes. Such syringes have the particularity to comprise a sheath protecting the needle, which can be retracted or extended. In a retracted position, the needle is completely covered and secured by the sheath. In an extended position, the needle projects outside the sheath.

A major issue when designing such safety syringes, further to securing the needle, is the ease of use of said safety syringes. Even though the protection sheath may enhance safety, its volume may make the syringe uneasy and cumbersome to manipulate.

Several safety syringes comprising a sheath have been proposed in the literature. However, none of them describes a sheath according to the invention, designed for an improved convenience and an improved safety for the healthcare worker.

For example, International Patent application WO 2006/105006 discloses a safety syringe including locking positions, formed by an inner sleeve receiving a cartridge filled with liquid, an outer sleeve through which said inner sleeve is telescopically reciprocated, and a plunger assembly that is attached to the inner sleeve and used to eject the liquid. This device is capable of both intermittent locking during use RU as well as permanent locking after use RAU (page 5, lines 2-3), performed by pushing inward a tab which engages permanently a hole. However, there is no specific retracted position before use RBU.

US patent 2011/0082428 relates to a safety structure for covering a syringe needle including a safety sleeve and a hub. The safety sleeve is fitted around the hub and provided with an axial sliding slot, a locating slot laterally extended from the sliding slot and a locating hole located at a bottom of the locating slot. The protective sheath comprises a "retracted position before use" in the locating slot, an "ejection position" in the sliding slot and a "retracted position after use" in the locating hole. However, this device does not provide a "retracted position during use". Moreover, this safety structure does not provide a one-way path for the male means of the hub through the female means of the safety sleeve: backtracks are indeed needed in order to access to the various retracted positions (RBU, RU and RAU). Thus said safety structure lacks of convenience and safety.

US patent US 4,994,045 discloses a safety syringe including a conventional syringe, a locating ring and an elongated and tubular sheath. This device is capable of both intermittent and permanent positions where the needle is secured, allowing the user to extend or retract the tubular sheath at will during use (ejection position E and retracted position during use RU), and to definitely secure the needle after use (retracted position after use RAU). However, the various retracted positions are not transversally aligned (see figure 2 of US 4,994,045), resulting in a non-optimized sheath length as described further in the description of the invention.

European Patent EP 1 603 612 discloses a preservative sheath for an injection needle or cannula arranged on a cannula-holding support which can be fitted onto a syringe. The sheath comprises guide means, which may be ramps, allowing the cannula to be in a retracted position before use RBU, a retracted position during use RU, a retracted position after use RAU and an ejection position E. However, the various retracted positions are not transversally aligned, resulting in a non-optimized sheath length as described further in the description of the invention. Moreover, the RU position comprises no temporary locking means.

US patent US 5,312,370 discloses a needle protecting device adapted for use on a standard syringe, especially a conventional dental syringe able to receive a cartridge. The sheath of this device may be rotated from an intermediate locked position during use RU to a final locked position after use RAU securing the needle. However, there is only one possible intermediate locked position (as described column 8 of US 5,312,370). This device provides no specific retracted position before use RBU.

Therefore, there is still a need in the art for a needle protective sheath improving the healthcare worker's convenience and safety.

### SUMMARY

**A first object of the present invention** is a protective sheath for a cannula arranged on a hub which can be fitted onto a syringe, said sheath comprising female means intended to cooperate with male means of the hub for guiding thereof, and characterized in that:
- said female means comprise a retracted position before use RBU, a retracted position during use RU, and a retracted position after use RAU, wherein the whole length of the cannula is inside said sheath;
- said female means comprise an ejection position E, wherein whole or part of the cannula protrudes out of said sheath; and
- at least two of the RBU, RU, and RAU positions are substantially transversally aligned.

Female means of the sheath according to the invention may include:
- primary transition means for insertion of male means of the hub within the sheath and for guiding said male means to the RBU position;
- primary locking means for temporary locking said male means in the RBU position;
- secondary transition means for guiding said male means from the RBU to the RU position;
- secondary locking means for temporary locking said male means in the RU position;
- guiding means, for guiding said male means forth from the RU position to the E position and back from the E position to the RU position;
- tertiary transition means for guiding said male means from the RU position to the RAU position; and/or
- final locking means, for permanently locking said male means in the RAU position.

In one embodiment, said male means are bosses. In one embodiment, female means are in the form of holes and/or grooves in the internal surface of the sheath.

Primary transition means may preferably be one-way transition means comprising anti-return means.

Secondary transition means may advantageously include translation/rotation guiding means from the RBU position to a transitory position T, and longitudinal guiding means from the T position to the RU position, said longitudinal guiding means being preferably one-way transition means comprising anti-return means.

In one embodiment, tertiary transition means may include rotational guiding means.

The sheath may further comprise anti-slipping means. For example, said anti-slipping means include inclined planes and/or at least one rib.

Anti-slipping means may further include means for preventing dismounting of the hub from the sheath, such as for example at least one rib.

The distal end of the sheath according to the invention may preferably be conical or beveled.

The sheath may further comprise gripping means, which are preferably ribs covered by a grinding or an erosion grain such as VDI 20 to 30, located on the external surface of the sheath.

Thus, the sheath according to the invention may comprise 1, 2, 3, 4 or all of the following features:
- female means are in the form of holes and/or grooves in the internal surface of said sheath cooperating with male means which may be bosses;
- said sheath comprises anti-slipping means, including:
   ∘ inclined planes and/or at least one rib; and/or
   ∘ at least one rib for preventing dismounting of the hub from said sheath;
- at least one end of said sheath is conical;
- said sheath comprises gripping means which are ribs covered by a grinding or an erosion grain such as VDI 20 to 30, located on the external surface of the sheath.

**A second object of the present invention is a hub** adapted to be inserted within a sheath as described above, comprising:
- male means, adapted to cooperate with female means of the sheath wherein the hub may be inserted; and
- optionally a cannula, protruding at both ends of the hub.

In an embodiment, the hub comprises reception means located at one end of the hub and designed to receive a syringe.

**A third object of the present invention** is an injection system, comprising a sheath as described above and a hub cooperating with said sheath, as described above. In one embodiment, the hub is inside the sheath.

**A fourth object of the present invention** is a syringe including a sheath or an injection system as described above.

The syringe of the invention may be a standard syringe comprising a plunger, a barrel or a carpule-holder and means for being adapted on a hub of the invention or an injection system of the invention. In an embodiment, the syringe is adapted for dentistry.

In an embodiment, the syringe of the invention is designed to hold a cartridge ; preferably the cartridge is immobilized in radial direction via demolding clips and the back of the syringe, and/or in axial direction at the neck of said cartridge via a clip which is preferably flexible.

The plunger of a syringe according to the invention may be advantageously equipped with a plunger seal comprising lips, wherein said lips may bend backwards for an easier introduction into the cartridge. According to one embodiment, the plunger seal is overmolded on the plunger.

The syringe may further comprise soft overmoldings installed in the thumb area and in the grip area of said syringe.

According to an embodiment, the syringe of the invention is disposable. In this embodiment, wherein the syringe is disposable, the syringe may have a precut located substantially at the distal end of the syringe, being for example a series of perforations or one or more circumferential grooves, preferably one circumferential groove.

According to an embodiment, the cartridge of said syringe is immobilized in radial direction via demolding clips and the back of the syringe; and the plunger of said syringe is equipped with a plunger seal comprising lips, wherein said lips may bend backwards for an easier introduction into the cartridge, said plunger seal being optionally overmolded on the plunger.

Thus, the syringe according to the invention may comprise 1, 2, 3 or all of the following features:
- the cartridge is immobilized in radial direction via demolding clips and the back of the syringe;
- the plunger of said syringe is equipped with a plunger seal comprising lips, wherein said lips may bend backwards for an easier introduction into the cartridge, said plunger seal being optionally overmolded on the plunger;
- said syringe comprises soft overmoldings installed in the thumb area and in the grip area of said syringe;
- said syringe has a precut located substantially at the distal end of the syringe, made of a series of perforations or one or more circumferential grooves, preferably one circumferential groove.

**A fifth object of the present invention** is a syringe designed to hold a cartridge, wherein the cartridge may be immobilized in radial direction via demolding clips and the back of the syringe, and/or in axial direction at the neck of said cartridge via a clip which is preferably flexible. In an embodiment, this syringe comprises a sheath of the invention, a hub of the invention or an injection system of the invention.

**A sixth object of the present invention** is a manufacturing device, preferably a mould or an assembly machine, for manufacturing a hub, a sheath, and/or a syringe according to the invention.

### DEFINITIONS

In the present invention, the following terms have the following meanings:
- "About" preceding a value means plus or less 10% said value.
- An **"anti-return"** means is here a means allowing the passage of a male means within a female means from a first position to a second position, and preventing the passage from the second position to the first position.
- **"Cannula"** refers to a tube that can be inserted into the body, generally for the delivery or removal of a fluid.
- **"Cartridge"** refers generally to a small sealed vial, used to contain and/or preserve a pharmaceutical composition.
- The hub is **"definitely locked"** within the sheath if said hub may move with regard to said sheath only if a brutal external force is applied, for example a force able to break the device.
- **"Distal end"** of a part refers to the farthest end of this part, relative to a point of origin. Most often the point of origin is the user and the distal end is the closest end to the tissue area in which the medicine will be injected.
- **"Distinct"** means physically separate. **"Distinct positions"** refer thus to physically or materially separate position.
- **"Ejection position"** refers here to a position of the hub when the cannula protrudes the sheath at least partially, rendering possible to insert the cannula in a patient for an injection for example.
- **"Female means"** refers to any cavity or hole in any shape, such as a groove, at the surface or through a material, cooperating with a male means. In other words, male means may be inserted into female means and may move inside.
- **"Hub"** refers to a piece capable of connecting a cannula to the distal end of a syringe according to the invention, or more generally to the distal end of any syringe, wherein the distal end of a syringe is the end farthest to the piston.
- The verb **"guiding"** used with male and female means, means that said female means provide a path via hole or grooves for instance, in which said male means may move freely, and prevent said male means to step out of said path, via walls or inclined planes for instance.
- **"Insertion"** of the male means refers here to the insertion of said male means within the female means of the sheath, said female means being possibly designed as to facilitate the insertion of said male means. For example, female means may be provided with a special opening in which male means are easily inserted. Insertion means may be also provided with an anti-return means, so that once inserted, male means cannot be removed from the female means.
- **"Integrally formed"** refers to a part in one piece, or to a part comprising several components, wherein any components making up the part have been rendered securely jointed.
- A **"locking means"** refers here to a part which prevents male means to step out of a defined position.
- **"Male means"** refers to any part constituted by matter, cooperating with female means. In other words male means may be inserted into female means and may move inside.
- A **"Position"** refers to a specific location within the female means of the sheath, wherein the male means are immobilized by for example sheath walls, locking means and/or transition means.
- **"Pharmaceutical composition"** refers to a composition comprising an active principle in association with a pharmaceutically acceptable vehicle or excipient. A pharmaceutical composition is for therapeutic use, and relates to health. Especially, a pharmaceutical composition may be indicated for treating or preventing a disease. According to the invention, the term **"treating a disease"** refers to reducing or alleviating at least one adverse effect or symptom of a disease, disorder or condition associated with a deficiency in an organ, tissue or cell function. The expression **"Preventing a disease"** or **"Inhibiting the development of a disease"** refers to preventing or avoiding the occurrence of symptom.
- **"Priming position"** refers to a position in which the user releases the air from the syringe and depresses the plunger until a small amount of the liquid medium comes out.
- **"Protective sheath"** or **"sheath"** refers here to a removable element preventing an easy access to another element able to hurt. It may be for example a tube preventing fingers to access a needle, which may be retracted or extended.
- **"Proximal end"** of a part refers to the closest end of this part, relative to a point of origin. Most often the point of origin is the user and the proximal end is the farthest end from the tissue area in which the medicine will be injected.
- **"Retracted position"** refers here to a position of the hub when the cannula is protected within the sheath, preventing fingers for example to access said cannula.
- **"Rotational movement"** refers here to the rotation of the hub within the sheath, around the axis of the cylinder.
- **"Standard syringe"** refers to a syringe comprising a barrel, wherein an injectable composition may be directly loaded.
- **"Substantially"** at the end of a part means that the location ranges from 0.01 to 25% of the total length of said part from said end.
- **"Syringe"** refers to any device comprising a container or a means intended to receive a container, an opening and a piston, wherein said piston is used to push the content of said container through said opening.
- The hub is here **"temporary locked"** within the sheath if the movement of said hub with regard to said sheath cannot spontaneously occur under common natural forces such as gravity, but requires an external force.
- **"Transition means"** refers to a path within the sheath allowing male means to step from a first position to a second position. A transition means may comprise an anti-return means so that said male means, once in the second position, cannot move back to the first position anymore.
- **"Translation movement"** refers here to a longitudinal movement, having the same direction than the axis of the cylinder.
- **"Transversally aligned"** refers here to the property of various points on a tube having a longitudinal axis X, wherein each point has the same coordinate along the X axis. **"Substantially transversally aligned"** refers here to the property of various points on a tube having a longitudinal axis X, wherein each point has the same coordinate plus or minus 3 mm, preferably plus or minus 2.5 mm, more preferably plus or minus 1.5 mm, even more preferably plus or minus 0.5 mm, still more preferably plus or minus 0.25 mm along the X axis.

### BRIEF DESCRIPTION OF THE DRAWINGS

**Figure 1** is a perspective view showing a portion of a sheath according to the invention, comprising locking, guiding and transition means.
**Figure 2** is a schema showing the various positions of the hub in the sheath, according to the invention.
**Figure 3** is a cross-sectional view of a sheath according to the invention, showing transition and locking means.
**Figure 4** is a partial cross-sectional view of the sheath according to the invention showing primary transition means.
**Figure 5** is a partial cross-sectional view of the sheath according to the invention showing secondary transition means and guiding means.
**Figure 6** is a cross-sectional view of a hub according to the invention.
**Figure 7** is a fragmentary cross-sectional view showing a hub, a portion of a sheath and a portion of a syringe according to the invention, said syringe being screwed into the hub and said hub being inserted into the sheath, wherein the sheath further comprises anti-slipping means.
**Figure 8** is a cross-sectional view showing a hub, a sheath and a portion of a syringe according to the invention, said syringe being screwed into the hub and said hub being inserted into the sheath.
**Figure 9** is a cross-sectional view showing a cartridge held by a self demolding clip inside a syringe according to the invention.
**Figure 10** is a cross-sectional view showing a cartridge held by the back of a syringe according to the invention.
**Figure 11** is a cross-sectional view showing a cartridge being inserted into a syringe according to the invention.
**Figure 12** is a cross-sectional view showing a cartridge inserted into a syringe according to the invention.
**Figure 13** is a cross-sectional view showing a plunger equipped with a vacuum plunger seal according to the invention, inserted into a cartridge.
**Figure 14** is a perspective front view of a cartridge syringe equipped with a sheath according to the invention.
**Figure 15** is a perspective view of a disposable syringe according to the invention, comprising a circumferential groove for a defined break.

**LIST OF REFERENCES**

| | | | |
|---|---|---|---|
| **100** | Sheath | **200** | Hub |
| **101** | Gripping means | **201** | Bosses |
| **102** | Distal end of the sheath | **202** | Distal end of the hub |
| **103** | Proximal end of the sheath | **203** | Proximal end of the hub |
| **104** | Internal surface of the sheath | **204** | Reception means of the hub |
| **105** | External surface of the sheath | **205** | Bottom of the reception means |
| **106** | Rib | **206** | Internal diameter of the hub |
| **107** | Rib | **207** | External diameter of male means |
| **110** | Primary transition means | **208** | Inclined plane |
| **111** | Hole | **209** | Gap |
| **120** | Primary locking means | **210** | Cannula |
| **121** | Inclined plane | **211** | Proximal end of the cannula |
| **130** | Secondary transition means | **300** | Syringe |
| **131** | Translation/rotation guiding means | **301** | Embossment |
| **132** | Longitudinal guiding means | **302** | Grip area |
| **133** | Inclined plane | **303** | Self demolding clip |
| **134** | Hole | **304** | Back of the syringe |
| **135** | Hole | **305** | Blocking means |
| **140** | Secondary locking means | **306** | Precut |
| **150** | Guiding means | **310** | Plunger |
| **151** | Inclined plane | **311** | Thumb area |
| **152** | Guiding means (second part) | **312** | Plunger seal |
| **160** | Tertiary transition means | **313** | Lips |
| **170** | Final locking means | **400** | Cartridge |
| **171** | Inclined plane | **401** | Rear piston of the cartridge |
| **172** | Inclined plane | **402** | Neck of the cartridge |

### DETAILED DESCRIPTION

### Protective sheath

The present invention relates to a protective sheath **100** for a cannula **210** arranged on a hub **200** which can be fitted onto a syringe **300,** allowing a safe use of said cannula **210** without the risk of being accidentally pricked.

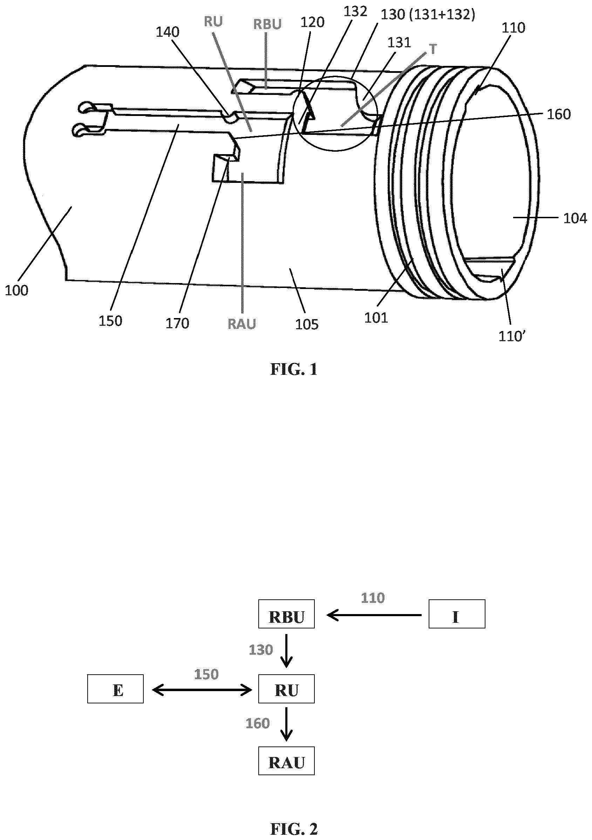

As shown in Figure 1, the sheath **100** has an overall shape of a hollow cylinder having a longitudinal axis X. In an embodiment, the sheath **100** has an internal diameter preferably ranging from 3 to 20 mm. In another embodiment, the sheath **100** has an external diameter preferably ranging from 4 to 21 mm. In one embodiment, the external and internal diameters of the sheath **100** are preferably substantially constant, at least in a first section. In one embodiment, a substantially constant diameter means that said diameter may vary of maximum 20%, preferably 10%, more preferably 5%, even more preferably 1%, from its nominal value (in other words the desired value).

In still another embodiment, the sheath **100** has a length preferably ranging from 30 to 150 mm.

In one embodiment, the sheath **100** is made of plastic, preferably transparent so that the cannula **210** may be visible through the sheath **100.** In another embodiment, the sheath **100** is opaque. In one embodiment, the thickness of the sheath **100** is preferably substantially constant (for example with a variation of maximum 20%, 10%, 5%, or 1% from its nominal value), but may be narrowed at non protective locations to save matter, for example at the distal and proximal ends **102** and **103** of said sheath **100.** In one embodiment, the thickness of the sheath **100** is comprised between 0.2 and 5 mm, preferably between 0.5 and 3 mm, more preferably between 1 and 2 mm, and said thickness may be narrowed of between 20 and 60% at non-protective locations.

The sheath **100** comprises female means which may cooperate with male means **201** of the hub **200,** said female means allowing said hub **200** to be in various positions, as depicted in Figure 2. In one embodiment female means cooperate with male means **201** of the hub **200,** said hub **200** being movable between the following positions:
- an insertion position I, wherein the hub **200** is introduced at the proximal end **103** of the sheath **100;**
- three retracted positions, wherein the hub **200** and the whole length of the cannula **210** are inside the sheath **100:**
   ∘ a retracted position before use RBU, wherein the hub **200** and the cannula **210** are temporary locked inside the sheath **100;**
   ∘ a retracted position during use RU, wherein the hub **200** and the cannula **210** are temporary locked inside the sheath **100;**
   ∘ a retracted position after use RAU, wherein the hub **200** and the cannula **210** are definitely locked inside the sheath **100;**
- an ejection position E, wherein the hub **200** is substantially at the distal end **102** of the sheath **100,** so that whole or part of the cannula **210** protrudes out of the sheath **100.** This E position is reachable from the RU position only.

The various positions of the hub **200** inside the sheath **100** contribute to the healthcare worker's safety, in that:
- the RBU position prevents needlestick injuries (NSIs) before use of cannula **210;**
- the RU position prevents NSIs during use of cannula **210;**
- the RAU position prevents NSIs after use of cannula **210.**

In a preferred embodiment, the insertion position, the retracted position before use, the retracted position during use, the ejection position and the retracted position after use are distinct one from another.

In another embodiment, the retracted position before use, the retracted position during use and the retracted position after use are distinct one from another.

In another embodiment, the retracted position before use and the retracted position during use are distinct.

In a preferred embodiment, the sheath **100** of the present invention includes at least 3 retracted positions (RBU, RU and RAU). In a preferred embodiment, the sheath **100** of the present invention includes more than 2 retracted positions. In an embodiment, the invention does not include any device featuring two retracted positions only.

In another embodiment, the sheath **100** does not provide a priming position distinct from the ejection position.

In an embodiment, the male means **201** is not temporary locked in the ejection position.

In an embodiment, the male means **201** is temporary locked in the ejection position.

The passage of male means **201** between the various positions and the immobilization of said male means **201** within the female means of the sheath **100** are rendered possible via:
- primary transition means **110,** for insertion of male means **201** of the hub **200** within the sheath **100,** and for guiding said male means **201** to the RBU position;
- primary locking means **120** for temporary locking said male means **201** in the RBU position;
- secondary transition means **130** for guiding said male means **201** from the RBU position to the RU position;
- secondary locking means **140** for temporary locking said male means **201** in the RU position;
- guiding means **150,** for guiding said male means **201** forth from the RU position to the E position and back from the E position to the RU position;
- tertiary transition means **160** for guiding said male means **201** from the RU position to the RAU position;
- final locking means **170,** for permanently locking said male means **201** in the RAU position.

Primary, secondary and tertiary transition means **110, 130** and **160** are preferably one-way transition means, meaning that they allow the transition from a first position to a second position only, but do not allow the reverse transition back from the second position to the first position. These transition means comprise therefore advantageously anti-return means. The achievement of a one-way path (i.e. without reverse transition) through the various retracted positions (RBU, RU and RAU) via the transition means (**110, 130,** and **160**) ensures an ease of use. In one embodiment, the sheath **100** contains female means which provide a precise continuous one-way path for the male means **201;** therefore avoiding routing errors and making use easier and safer. In one embodiment, the sheath **100** contains female means which provide a precise one-way path for the male means **201;** therefore avoiding routing errors and making use easier and safer.

In one embodiment, the guiding means **150** is the only means which allows reverse transition. It is obvious from one skilled in the art that the guiding means **150** to the ejection position has to allow reverse transition.

The various positions, being successively reached, prevent a cannula **210** equipped with said sheath **100** to be re-used.

A major drawback of using a protective sheath on a cannula is the reduction of manipulation freedom during operation, due to the volume of said sheath.

It has been therefore a goal for the present invention to provide a sheath as short as possible in order to facilitate manipulation during operation, but long enough to ensure a safe manipulation of the device.

A safe manipulation of a cannula **210** inserted into a sheath **100** is obtained when safety lengths between the sharp edges of the cannula and the sheath ends are respected (at both distal and proximal end **102** and **103**)**.** The sheath **100** has therefore to be as long as the cannula length plus the safety lengths. Safety length depends on the diameter of the opening at the sheath end, from where a cannula end may be accessed. Typically, a safety length of between 1 and 5 mm, preferably about 3 mm, has to be respected if the diameter of the opening at the sheath end is of about 10 mm.

The present invention provides a protective sheath **100** in which the proximal and distal portions of the cannula **210** are protected. For specific applications, the cannula **210** arranged on the hub **200** has to exceed the proximal end of the hub **203.** For instance in dentistry, the proximal end of the cannula **211,** protruding from the proximal end of the hub **203,** perforates the carpule hold in the barrel of a syringe when said syringe is inserted inside the proximal part of the sheath **103.** Therefore it is necessary to protect the user from the proximal end of the cannula **211.** In the present invention the RBU, RU and RAU positions are positioned such as the proximal end and the distal end of the cannula **210** are contained inside the sheath **100.**

In one embodiment of the present invention, the cannula **210** may be totally contained inside the sheath **100.** Especially, in the invention, the protection of the cannula **210** is granted only by the sheath **100** and not by the hub **200.**

In one aspect, the present invention aims at protecting healthcare worker from needlestick injuries. In an embodiment, the sheath does not to prevent a patient from seeing the needle prior to an injection. In another embodiment, the sheath prevents a patient from seeing the needle prior to an injection.

The Applicant found that, when the various retracted positions RBU, RU and RAU are transversally aligned, wherein the three positions are in the same cross-section, the sheath length is optimized. Aligned retracted positions allow the sheath to be the shortest possible while effectively protecting the cannula.

The provision of a short sheath results in a full adaptability of the sheath on the smallest syringes on market when still using a long cannula length.

Thus, the present invention provides a sheath **100** having an optimized sheath length, wherein at least two of the RBU, RU and RAU positions, preferably all of the RBU, RU and RAU positions are substantially transversally aligned. In a preferred embodiment, the RBU, RU and RAU are substantially transversally aligned. In another embodiment, the RBU and RU are substantially transversally aligned. In another embodiment, the RU and RAU are substantially transversally aligned. In another embodiment, the RBU and RAU are substantially transversally aligned. In one embodiment, the transversal alignment is respected for the aligned positions with a tolerance of 6 mm, preferably 5 mm, more preferably 3 mm, even more preferably 1 mm, still more preferably 0.5 mm. In other words, the absolute difference between the smallest and the greatest coordinate of the different aligned positions along the X axis does not exceed the value of said tolerance.

As disclosed in Figure 2, the female means of the sheath **100** provides a one way path for the male means **201** from the RBU position to the RAU position through the RU position. The female means of the sheath **100** also allows the male means to go from the RU position to the E position and conversely.

In one embodiment, the male means **100** moves transversally respectively from the RBU position to the RU position and then to the RAU position.

In one embodiment, the male means **100** moves transversally respectively from the I position to the RBU position, the RU position and then to the RAU position.

In one embodiment, the male means **100** moves transversally respectively from the I position to the RBU position, the RU position, the E position, the RU position and then to the RAU position.

In order to better secure the use of a cannula **210** arranged on a hub **200,** the transition of male means **201** of said hub **200** from a RBU position to a RU is advantageously performed via two steps:
- a displacement of said hub **200** within the sheath **100** toward the proximal end **103** of said sheath **100;**
- a displacement of said hub **200** within the sheath **100** toward the distal end **102** of said sheath **100.**

The two-step transition between the RBU position to the RU position results in improved safety, where the cannula **210** arranged on the hub **200** inserted into the sheath **100** is less likely to protrude said sheath **100** hazardously.

Thus, in one embodiment, secondary transition means **130** includes translation/rotation guiding means **131** for guiding male means **201** from the RBU position to a transitory position T, and longitudinal guiding means **132** for guiding said male means **201** from the T position to the RU position.

In one embodiment, longitudinal guiding means **132** are one-way transition means comprising anti-return means.

In an embodiment, tertiary transition means **160** are actuated via a rotational movement of the hub **200** inside the sheath **100,** preferably via an unscrewing movement. This feature combines the entering in the final lock position of the sheath **100** with the natural gesture of the user in cannula **210** end life management. Thus, if a syringe **300** is screwed in the hub **200,** the transfer into the final locked position RAU has the advantage to be automatic when the user unscrews the syringe **300.**

Thus, in one embodiment, tertiary transition means **160** include rotational guiding means.

In a preferred embodiment, the final lock position of the sheath **100** can be reached only from the RU position and not from the RBU position. In another embodiment, the male means **201** moves from the ejection position to the RAU position only through the RU position. In another embodiment, the male means **201** moves from the ejection position to the RAU position without passing through the RBU position. Moreover, the male means **201** reaches the RU position from longitudinal translation guiding means **132** and not from rotational transition means, thus avoiding routing errors as guiding means **150** (used in one of the following steps, cf. Figure 2) comprises also a longitudinal groove in the axis of the transition means **132.** In one embodiment, the guiding means **150** is in line with the longitudinal translation guiding means **132.**

In an embodiment, the secondary locking means **140** is compulsory between the RU position and the ejection position E.

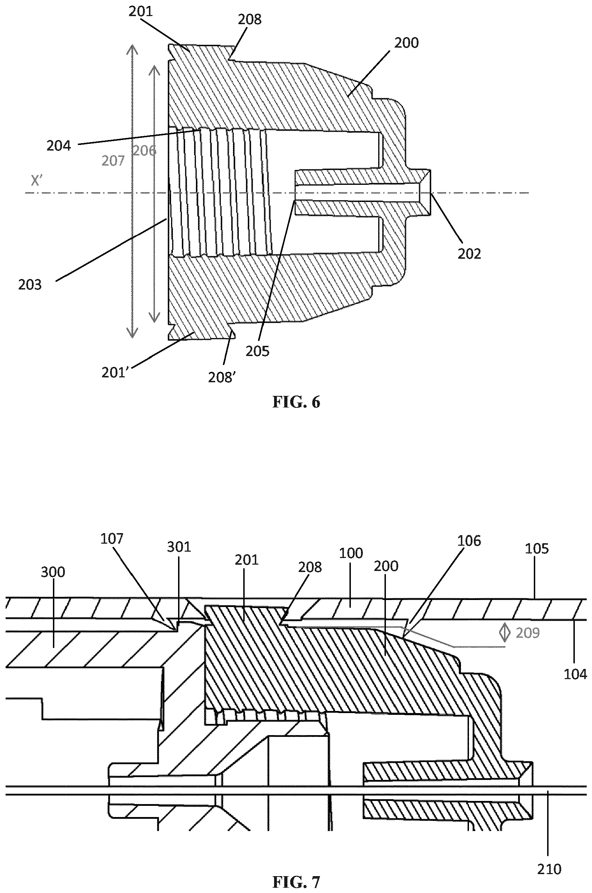

For the efficacy of the product, the hub **200** has to remain in the sheath **100.** Unfortunately, as a result of the fabrication process, a gap **209** between the sheath **100** and the hub **200** in the locking area cannot be avoided, as described Figure 7. Thus, a risk exists that male means **201** on the hub **200** slip under the sheath **100,** which eliminates the safety feature of the device.

Therefore, for safety improvement, the sheath **100** according to the invention may further comprise anti-slipping means.

In one embodiment, anti-slipping means include inclined planes **208** at the ends of male means **201.** In one embodiment, anti-slipping means include inclined planes at strategic locations **121, 133, 171** and **172** on the walls of the sheath **100,** as described in Figures 1, 3, 4 and 5. In one embodiment, anti-slipping means include inclined planes **208** at the ends of male means **201** and at strategic locations **121, 133, 171** and **172** on the walls of the sheath **100,** as described in Figures 1, 3, 4 and 5. The angles of the inclined planes are such that when forces are applied in an axial direction, forces perpendicular to the axis force the sheath **100** to get narrower to the hub **200.** The angles range preferably from 30° to 60°, more preferably from 40° to 50°, even more preferably about 45°.

Therefore, the slipping of the hub **200** under the sheath **100** is avoided, and the safety is improved.

In an embodiment, anti-slipping means further include at least one rib **106,** located just next to any hole or groove of the sheath **100** toward the distal end **102** of said sheath **100** so that male means **201** are not likely to pass across said rib **106** if a slipping of the hub **200** within the sheath **100** occurs (figure 7).

In an embodiment, anti-slipping means include means for preventing dismounting of the hub **200** from the sheath **100.** Although proper use of the device is supposed, the misuse of the device has also to be taken into consideration. In this embodiment, at least one rib **107** may be added, in combination with an embossment **301** present on the hub **200** or near the distal end of the syringe **300,** as depicted in Figure 7. This rib **107** is advantageously designed so that once the hub **200** is inserted in the sheath **100** and is passed across said rib **107,** this rib **107** prevents said hub **200** to step back, acting thus as a non-dismountable feature. This rib **107** may be installed near the proximal end of the sheath **100,** for example in the first 4 cm, 3cm, 2 cm or 1 cm on the internal surface **104** of said sheath **100.**

Still for a safety improvement, the distal end **102** of the sheath **100** is preferably conical or beveled. This shape results on providing a large opening for a cannula **210** which may be inadvertently bended, and in being tight enough at the distal end to avoid the fingers to access said cannula **210.** In one embodiment, the proximal end **103** of the sheath **100** is not conical or beveled in order to easy the insertion of the distal end of a syringe **300.** In an embodiment, the protective sheath **100** has a length adapted to protect the user from needlestick.

In one embodiment, the distal end **102** of the sheath **100** does not comprise any additional piece, such as a plug, to prevent the hub **200** to step out of the sheath **100.**

In one embodiment, the sheath **100** further comprises gripping means **101,** providing a good grip toward the gloves of healthcare workers. Said gripping means **101** may be for example ribs, preferably covered by a grinding or an erosion grain like VDI 20 to 30.

As shown in Figures 1, 3, 4 and 5, all transition and locking means may be provided with holes, grooves, inclined planes and/or lugs, arranged in two identical systems diametrically opposite on the sheath **100** regarding the axis X, so that one system allows the movement and the locking of a first male means **201** of the hub **200** and the second system allows the movement and the locking of a second male means **201'** of the hub **200.** Said male means **201** and **201'** are moved by pushing, pulling and/or rotating with one hand a syringe **300** engaged in the hub **200,** and by holding the sheath **100** with the other hand.

More than two systems are possible, for example three, four, five or six systems. In all cases, all systems are preferably identical and regularly spaced along a path centered on the axis X.

In one embodiment, the sheath **100** of the present invention comprises female means which are in the form of holes and/or grooves in the internal surface **104** of said sheath **100.**

In another embodiment, the sheath **100** of the present invention comprises anti-slipping means, including inclined planes **121, 133, 171, 172** and/or at least one rib **106;** and/or at least one rib **107** for preventing dismounting of the hub **200** from said sheath **100.**

In another embodiment, the sheath **100** of the present invention comprises:
- anti-slipping means, including:
   ∘ inclined planes **121, 133, 171, 172** or at least one rib **106;** or
   ∘ at least one rib **107** for preventing dismounting of the hub **200** from said sheath **100.**

In another embodiment, the sheath **100** of the present invention comprises:
- anti-slipping means, including:
   ∘ inclined planes **121, 133, 171, 172** and at least one rib **106;** or
   ∘ at least one rib **107** for preventing dismounting of the hub **200** from said sheath **100.**

In another embodiment, the sheath **100** of the present invention comprises:
- anti-slipping means, including:
   ∘ inclined planes **121, 133, 171, 172** and at least one rib **106,** and
   ∘ at least one rib **107** for preventing dismounting of the hub **200** from said sheath **100.**

In another embodiment, the sheath **100** of the present invention comprises a conical or beveled distal end.

In another embodiment, the sheath **100** of the present invention comprises gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;** and
- anti-slipping means, including:
   ∘ inclined planes **121, 133, 171, 172** and/or at least one rib **106;** and/or
   ∘ at least one rib **107** for preventing dismounting of the hub **200** from said sheath **100.**

In another embodiment, the sheath **100** of the present invention comprises:
- female means which are in the form of holes and/or grooves in the internal surface **104** of said sheath **100;** and
- a conical or beveled distal end.

In another embodiment, the sheath **100** of the present invention comprises:
- female means which are in the form of holes and/or grooves in the internal surface **104** of said sheath **100;** and
- gripping means **101** which are ribs covered by a grinding or an erosion grain such as VDI 20 to 30, located on the external surface **105** of said sheath **100.**

In another embodiment, the sheath **100** of the present invention comprises:
- anti-slipping means, including:
   ∘ inclined planes **121, 133, 171, 172** and/or at least one rib **106;** and/or
   ∘ at least one rib **107** for preventing dismounting of the hub **200** from said sheath **100;** and
- a conical or beveled distal end.

In another embodiment, the sheath **100** of the present invention comprises:
- anti-slipping means, including:
   ∘ inclined planes **121, 133, 171, 172** and/or at least one rib **106;** and/or
   ∘ at least one rib **107** for preventing dismounting of the hub **200** from said sheath **100;** and
- gripping means **101** which are ribs covered by a grinding or an erosion grain such as VDI 20 to 30, located on the external surface **105** of said sheath **100.**

In another embodiment, the sheath **100** of the present invention comprises:
- a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
   - anti-slipping means, including inclined planes **121, 133, 171, 172** and/or at least one rib **106;** and
   - a conical or beveled distal end.

In another embodiment, the sheath **100** of the present invention comprises:
- female means which are in the form of holes and/or grooves in the internal surface **104** of said sheath **100;**
   - anti-slipping means, including at least one rib **107** for preventing dismounting of the hub **200** from said sheath **100;** and
   - a conical or beveled distal end.

In another embodiment, the sheath **100** of the present invention comprises:
- female means which are in the form of holes and/or grooves in the internal surface **104** of said sheath **100**;
- anti-slipping means, including:
   ∘ inclined planes **121, 133, 171, 172** and/or at least one rib **106;** and
   ∘ at least one rib **107** for preventing dismounting of the hub **200** from said sheath **100;** and
      - a conical or beveled distal end.

In another embodiment, the sheath **100** of the present invention comprises:
- female means which are in the form of holes and/or grooves in the internal surface **104** of said sheath **100;**
- anti-slipping means, including inclined planes **121, 133, 171, 172** and/or at least one rib **106;** and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
- anti-slipping means, including at least one rib **107** for preventing dismounting of the hub **200** from said sheath **100;** and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
- anti-slipping means, including:
   ∘ inclined planes **121, 133, 171, 172)** and/or at least one rib **106;** and
   ∘ at least one rib **107** for preventing dismounting of the hub **200** from said sheath **100;** and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100**;
   - a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- anti-slipping means, including at least one rib **107** for preventing dismounting of the hub **200** from said sheath **100**;
- a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- anti-slipping means, including inclined planes **121, 133, 171,** 172 and/or at least one rib **106;**
- a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- anti-slipping means, including:
   ∘ inclined planes **121, 133, 171, 172** and/or at least one rib **106;** and
   ∘ at least one rib **107** for preventing dismounting of the hub **200** from said sheath **100;**
- a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
- anti-slipping means, including inclined planes **121, 133, 171, 172** and/or at least one rib 106;
   - a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
- anti-slipping means, including at least one rib **107** for preventing dismounting of the hub **200** from said sheath **100;**
- a conical or beveled distal end; and
- gripping means **101** which are ribs covered by a grinding or an erosion grain such as VDI 20 to 30, located on the external surface **105** of said sheath **100.**

In another embodiment, the sheath **100** of the present invention comprises:
- female means which are in the form of holes and/or grooves in the internal surface **104** of said sheath **100;**
- anti-slipping means, including:
   ∘ inclined planes **121, 133, 171, 172** and/or at least one rib **106;** and
   ∘ at least one rib **107** for preventing dismounting of the hub **200** from said sheath **100;**
- a conical or beveled distal end, and
- gripping means **101** which are ribs covered by a grinding or an erosion grain such as VDI 20 to 30, located on the external surface **105** of said sheath **100.**

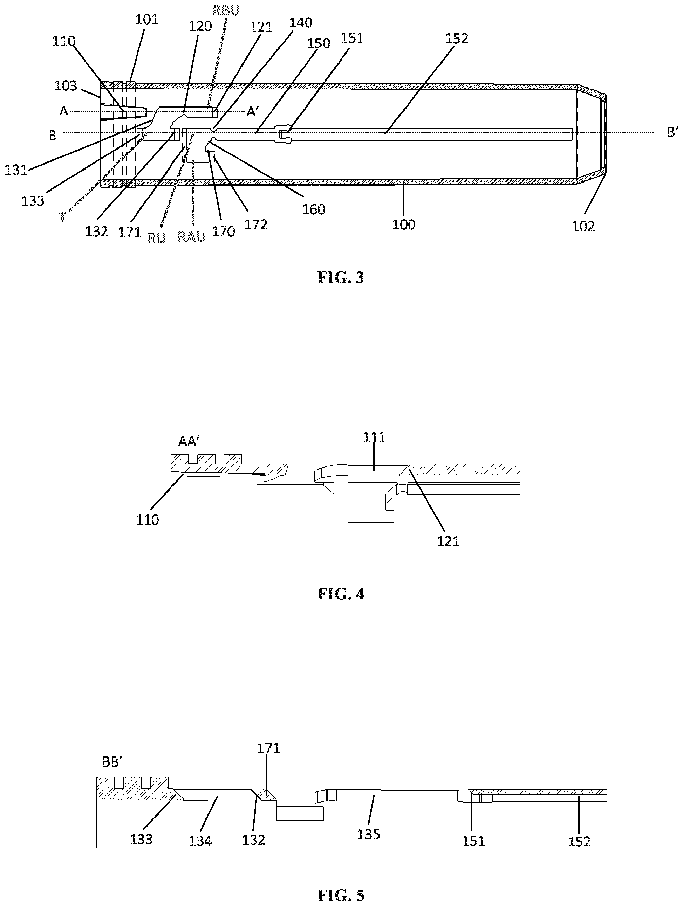

A primary transition means **110** is made by scooping out the interior surface of the sheath **100** to obtain a groove opening only to the internal surface of said sheath **100,** which ends at its distal end by an inclined plane directed to the interior of said sheath **100,** leading thus the depth of said groove to a null value (Figure 4). Said primary transition means **110** comprises a hole **111** which opens both to the internal and external surfaces **104** and **105** of said sheath **100,** located further to the inclined plane toward the distal end **102** of said sheath **100.** The width of the groove of said primary transition means **110,** as illustrated in Figure 3, has a width increasing gradually as one approaches the proximal end **103** of the sheath **100.** The function of this feature is to facilitate the insertion of male means **201** of the hub **200** in the sheath **100.**

A primary locking means **120** comprises a lug, located between two holes opening to both internal and external surfaces **104** and **105** of the sheath **100.** This lug, in complement to the walls of the sheath **100** which prevent male means **201** to go further toward the distal end **102** of the sheath **100,** maintains said male means **201** in the RBU position.

A secondary transition means **130** comprises two guiding means:
- a translation/rotation guiding means **131,** and
- a longitudinal translation guiding means **132.**

A translation/rotation guiding means **131** consists in a hole (represented as **111** and **134**) and a wall of the sheath **100,** being designed as to present a curved and smooth shape so that when longitudinally moving male means **201** from the RBU position toward the proximal end **103** of the sheath **100,** said male means **201** slightly rotate to move to the T position (Figure 3).

A longitudinal translation guiding means **132** is made by an inclined plane directed toward the interior of the sheath **100,** located next to the T position regarding the X axis. Said longitudinal guiding means **132** comprises at its proximal and distal ends holes **134** and **135** opening both to the external and internal surface of the sheath **100,** as described in Figure 5. By longitudinally moving male means **201** toward the distal end **102** of the sheath **100** from the T position, said male means **201** are pushed inside the sheath **100** by the guiding means **132** and emerge in the RU position just next to said guiding means **132.** A guiding means **132** is advantageously a one-way transition means.

The slope of the inclined planes directed toward the interior of the sheath **100** ranges preferably from 10% to 30%, more preferably of about 20%, to promote smooth transition from one position to another.

A secondary blocking means **140** is made by a lug, configured so that male means **201** may be maintained in longitudinal distal abutment in the position RU. Male means **201** are further radially maintained in the position RU by on the one hand a wall of the sheath **100,** and on the other hand tertiary transition means **160.** The secondary blocking means **140** is designed so that the passage forth from the position RU to the position E and back from the position E to the position RU requires a force high enough to push the lug apart.

A guiding means **150** comprises a longitudinal groove which opens to both internal and external surface **104** and **105** of the sheath **100** in a first part, and to only the internal surface **104** of said sheath **100** in a second part **152.** Between these two parts, a guiding means **150** comprises an inclined plane **151** directed toward the interior of the sheath **100,** to allow a smooth transition from the first part of the groove to the second part **152.** The second part **152** of the groove ends up substantially at the distal end **102** of the sheath **100,** approximately at the beginning of the conical distal end of said sheath **100.** Male means **201** may be stopped by the end of the guide means, and/or optionally by the return of the sheath wall inside the sheath **100,** at its distal end **102.**

The groove of the guiding means **150** preferably opens to the internal surface **104** and not to the external surface **105** of the sheath **100.** This feature provides reinforcement for the guiding of male means **201** with the matter which covers the bottom of the grooves and which forms the grooves. However, it is possible to make the grooves open to both internal and external surfaces **104** and **105** of the sheath **100,** or to make the grooves open to both internal and external surfaces **104** and **105** in a first part and open to only the internal surface **104** in a second part.

A tertiary transition means **160** is made by a lug having an inclined plane so that male means **201** may move from the position RU to the position RAU, via a force high enough to put said lug apart. Once in the position RAU, the lug comprises a surface **170** preventing said male means **201** to step back. Thus, the lug, via its surfaces **160** and **170,** is both a transition and locking means.

In the position RAU, male means **201** are further immobilized via the walls of the sheath **100.** The hub **200** is therefore definitely locked in the RAU position, and cannot go forward or backward with regard to the sheath **100.**

### Injection system

The present invention further relates to an injection system, comprising a sheath **100** and a hub **200,** inserted within said sheath **100.**

The hub **200** has an overall shape of a cylinder having a longitudinal axis X', as described in Figure 6. The hub **200** is open at its proximal end **203** and is closed at its distal end **202** on the cannula **210** or on a cylindrical cavity for the reception of the cannula **210.** The hub **200** comprises a reception means **204** at its proximal end **203,** designed to receive the distal end of a syringe **300,** which is the end farthest to the thumb area **311.**

In one embodiment, the reception means **204** is a thread allowing a clipping or a screwing of the distal end of a syringe **300.** This thread may be sectioned by two longitudinal slots, for ensuring a clipping by pulling away the two threaded parts forming each a jaw, during the positioning of the hub **200** on the syringe distal end.

In one embodiment, wherein the hub comprises a cannula **210,** said cannula **210** protrudes both distal end **202** and the bottom **205** of the reception means **204.** When the distal end of a syringe **300** is engaged in the reception means **204,** the cannula **210** is thus in fluid communication with the content of said syringe **300** via its proximal end **211.** In other words, the content of the syringe **300** may be flowed through the cannula **210** from the proximal end **211.** The content of said syringe **300** may be for example a pharmaceutical composition, which may be contained within a cartridge **400** or the barrel of said syringe **300,** depending on the syringe type. In another embodiment the cannula may comprise two separate parts (i.e. two cannulae) one protruding outside of the distal end of the hub and one protruding outside of the proximal end of the hub; said two cannulae being fluidly connected inside the hub.

In an embodiment, the hub is used to move between the various position implemented in the sheath, to hold the cannula and, optionally, to fluidly connected the cannulae; however the hub is not design to protect the user from the cannula.

The hub **200** has an external diameter **206** slightly inferior to the diameter of the internal surface **104** of the sheath **100.**

In one embodiment, the hub **200** further comprises at least one, or two, three, four, five or six; preferably two male means **201** diametrically opposite regarding the axis X'. In one embodiment, the hub **200** further comprises at least one male means **201.** In a preferred embodiment, the hub **200** further comprises at least two male means **201** diametrically opposite regarding the axis X'. In another embodiment, the hub **200** further comprises at least three male means **201** regularly spaced on the circumference of the hub **200.** In another embodiment, the hub **200** further comprises at least four male means **201** regularly spaced on the circumference of the hub **200.** In another embodiment, the hub **200** further comprises at least five male means **201** regularly spaced on the circumference of the hub **200.** In another embodiment, the hub **200** further comprises at least six male means **201** regularly spaced on the circumference of the hub **200.** At these male means, the diameter **207** is greater than the diameter of the internal surface **104** of the sheath **100,** and preferably substantially identical (permitting a variation of 10%, 5%, or 1% for example), or slightly inferior, to the diameter of the external surface **105** of the sheath **100.**

In an embodiment, the hub **200** does not contain a U-shaped channel or groove around the male means **201.**

The hub **200** is intended to be introduced at the proximal end **103** of the sheath **100,** so that the axes X and X' line up. Male means **201** are thus advantageously adapted to be inserted in female means of the sheath **100.**

In one embodiment, male means **201** of the hub **200** are outwards projections such as bosses.

In one embodiment, male means **201** of the hub **200** are bosses.

In an embodiment, the injection system of the invention comprises a sheath **100** and a hub **200** inserted in said sheath **100,** wherein male means **201** are in a RBU position.

In an embodiment, the injection system of the invention is delivered to the user with the male means **201** in a RBU position.

In an embodiment, the sheath **100** of the present invention is not self-deploying.

The I position is thus used during the manufacturing and assembly of the injection system of the invention.

### Syringe

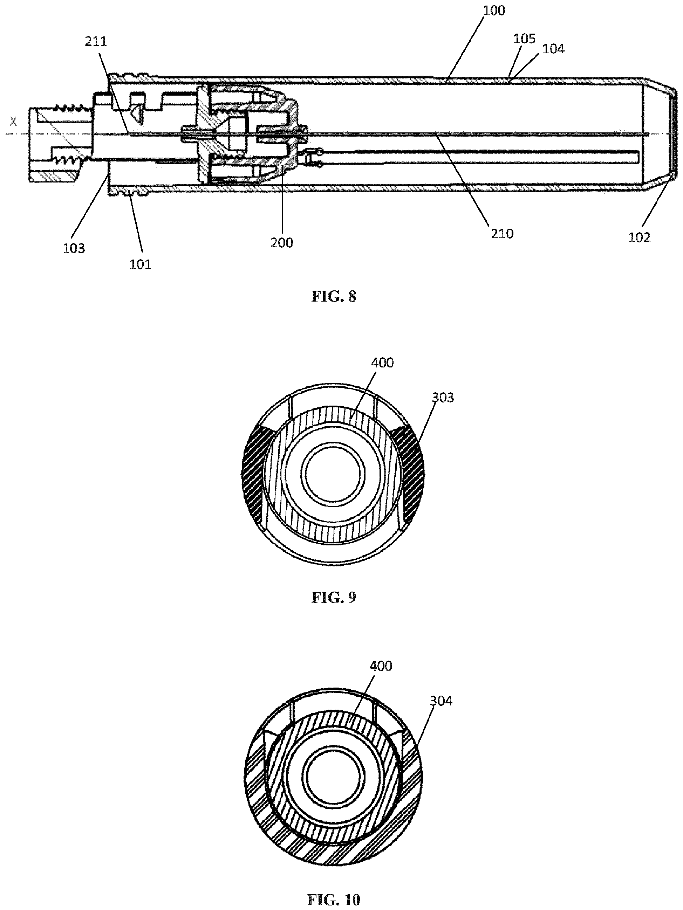

This invention also relates to a syringe **300,** preferably to a dental syringe for the injection of a pharmaceutical composition, for instance, of a local anesthetic agent, comprised within a cartridge **400** or the barrel of said syringe **300;** said syringe **300** being equipped with a sheath **100** protecting an injection needle or cannula **210** arranged on a hub **200,** or being equipped with an injection system according to the invention comprising a sheath **100,** a hub **200** and a cannula **210** (Figure 8).

In one embodiment, no other piece than the sheath **100** is intended to protect the cannula **210:** in other words, the sheath **100** is the only safety part for the protection of the cannula **210.**

In one embodiment, no spring is involved in the protection of the cannula **210,** for example in an automatic passage from an extended position to a retracted position, preventing thus a hazardous release of said spring.

The hub **200** is intended to be positioned at the distal end of the syringe **300,** and is aimed at ensuring the entry of the proximal end **211** of the cannula **210** within the cartridge **400** or barrel containing the pharmaceutical composition. In one embodiment, the hub **200** is screwed on the distal end of the syringe **300.**

In another embodiment, the hub **200** and the syringe **300** are integrally jointly formed.

In one embodiment, the syringe **300** is a standard syringe.

In another preferred embodiment, the syringe **300** is designed to hold a cartridge **400.**

In the embodiment, wherein the syringe **300** is designed to hold a cartridge **400,** said syringe is equipped with at least one means to hold a cartridge **400.** Preferably, the syringe **300** is equipped in a first section of a first means to hold a cartridge **400,** and is equipped in a second section of a second means to hold said cartridge **400,** said means being complementary to safely hold said cartridge **400.**

In one embodiment, first means to hold the cartridge **400** are self demolding clips **303,** presenting a protrusion to hold said cartridge **400,** said protrusion being flexible enough to allow the insertion of said cartridge **400** (Figure 9).

In one embodiment, the second means to hold the cartridge **400** is the bottom of the syringe **304,** as described in Figure 10.

By these means, the cartridge **400** is totally immobilized in radial direction once inserted into the self demolding clip, avoiding thus to fall out of the syringe **300.**

The cartridge **400** is immobilized in axial direction via a blocking means **305.** An axial immobilization is of great importance for the present invention, since active aspiration is therefore allowed by suction of the rear piston **401** of the cartridge **400.** This is particularly useful in dental care, during anesthesia for instance. The dentist loads a cartridge containing the anesthetic agent in a syringe and inserts then the needle tip into the gum. If the needle tip is inserted into a blood vessel, a depression will immediately aspirate blood into the cartridge. The dentist, in order to avoid an injection of anesthetics into a blood vessel, will be averted by the change of the anesthetic color.

Instead of axially fixing the rear of the cartridge **400** as it is the case in common syringes, the cartridge **400** is advantageously maintained at its neck **402,** so that the length of the cartridge **400** does no longer play a role in the aspiration process. The syringe of the invention is therefore adaptable on cartridges having different lengths.

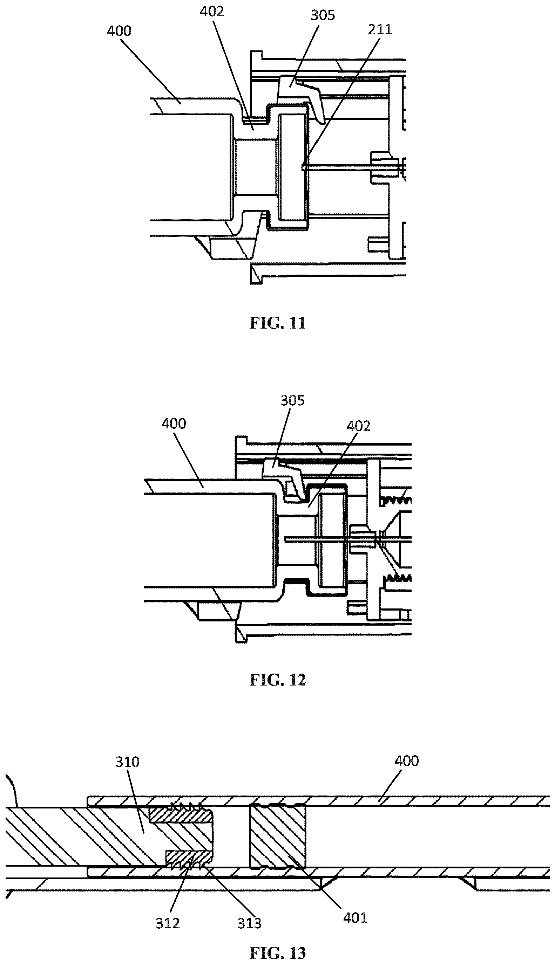

In one embodiment, the blocking means **305** is a clip, as shown in Figure 11. Preferably, the blocking means **305** also immobilizes the cartridge **400** radially.

The blocking means **305** is advantageously flexible, allowing the cartridge insertion. When the cartridge **400** is pushed into the end position, the cartridge head will push the blocking means **305** up (Figure 11). Once the cartridge **400** is in place, the blocking means **305** drops back and holds the cartridge **400** at its neck **402,** immobilizing thus said cartridge **400** for active aspiration (Figure 12).

An advantage of using such a flexible blocking means is the provision of passive aspiration. The blocking means **305** may indeed be slightly pushed upward during each pressure on the plunger **310** of the syringe **300,** and will then come back to its initial position creating a depression in the cannula **210.** Preferably, the blocking means **305** allows the cartridge **400** to travel between 1 and 5 mm, more preferably between 2 and 4 mm, to assure passive aspiration.

During active aspiration, the plunger **310** of the syringe **300** may be provided with a fixation connecting said plunger to the rear piston **401** of the cartridge **400** to assure the pulling of said rear piston of said cartridge. Such a fixation is for example a barb or a harpoon, or any other means adaptable to the rear piston of a cartridge.

In a preferred embodiment, the syringe plunger **310** is able to create a depression in the cartridge **400,** aspirating thus the cartridge rear piston **401** and causing a depression in the cannula **210.**

In this embodiment, the syringe plunger **310** is preferably provided with a plunger seal **312,** as described in Figure 13.

The plunger **312** seal creates a depression between the syringe plunger **310** and the rear piston **401** of the cartridge **400** when the user pulls on the syringe piston, which results in an aspiration in the cartridge **400.**

The plunger seal **312** comprises advantageously lips which may bend backwards (i.e. towards the proximal part of the plunger), allowing an easier introduction into the cartridge **400** without destroying the seal area. When pushing the plunger **310** in the cartridge **400,** the air between the cartridge rear piston **401** and the vacuum plunger seal **312** expels since the lips can fold back, allowing the air to leave the space.

Whereas the fixation of a plunger to the rear piston of a cartridge has generally to be adapted in function of the cartridge type, an advantage of using such a plunger seal **312** is the adaptability on several types of cartridge.

In one embodiment, the vacuum plunger seal **312** is overmolded on the plunger **310.**

Chirurgical instruments are manipulated with latex gloves. During the operation the gloves get wet and the friction forces of the gloves rubbers are reduced.

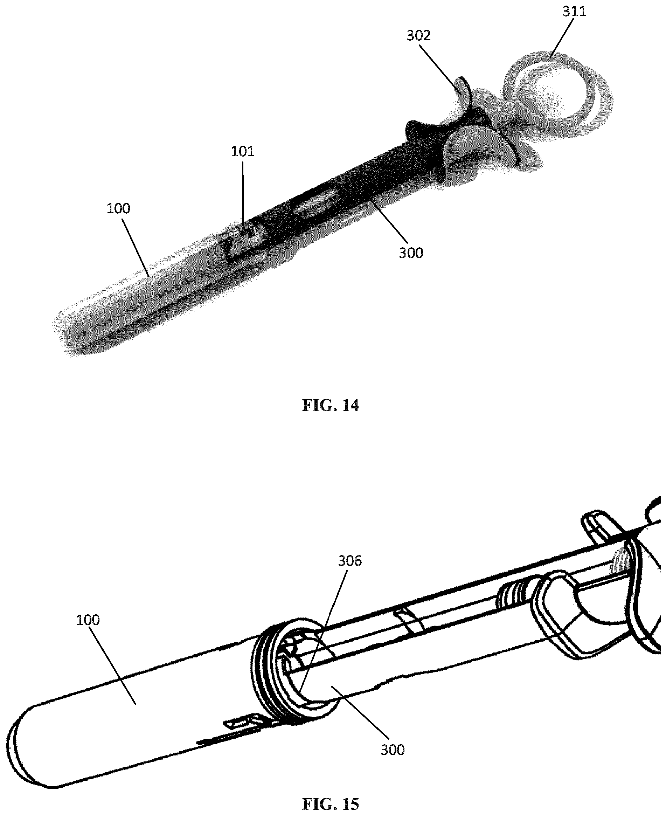

When using a plastic part with wet gloves the finger slipping could introduce injuries to the patient. In order to increase the grip, soft overmolding may be installed in the thumb area **311,** and in the grip area **302** of the syringe **300,** as depicted Figure 14. A surface treatment like erosion graining VDI 20 to 30 or surface grindings, nobs or ribs may also be used to increase the surface friction.

Material for these grips may be for example SEBS, TPU, TPE, preferably SEBS (Kraiburg Thermoplast W, Soloplast TH, etc.).

In combination with the gripping means **101** of the sheath **100,** the syringe of the invention provides a comfortable and safe use.

In one embodiment, the syringe of the invention is a disposable syringe, and allows preferably only a single use.

In this embodiment, the syringe **300** may have the particularity to be conveniently broken after use, in order to separate the sticking part from the non-sticking part. In one embodiment, the sticking part comprises the cannula **210** covered by the sheath **100,** the sheath **100,** the hub **200** and a optionally the distal end of the syringe **300** which is engaged in the reception means **204** of the hub **200,** and the non-sticking part comprises substantially the totality of the syringe **300** which may comprise a cartridge **400.**

This has the advantage to reduce the volume of sticking waste having a high risk of contamination, which has to be withdrawn in special containers whose decontamination and destruction are cost intensive. Another advantage is to avoid the congestion of the low volume waste boxes used by medical personal operating in reduced space.

Further to make the end of life of the syringe comfortable, another advantage of such a breakable syringe is the increased difficulty to uncover the cannula **210** after use. The cannula **210** may not be accidentally pushed out of the sheath **100** and become exposed, rendering a needlestick injury even less likely to happen.

A means for an easy and defined breakage is for example a precut **306,** made of a series of perforations, or one or more circumferential grooves, located substantially at the distal end of the syringe **300,** for example in the last 3 cm, 2 cm, 1 cm or 5 mm of said syringe **300.** Preferably, a means for an easy and defined breakage is a circumferential groove, as depicted in Figure 15.

In the embodiment wherein the syringe **300** is disposable, and wherein the final locked position for the hub **200** in the sheath **100** is reached via an unscrewing movement, the invention provides a really convenient end of life by an unscrewing movement followed by a break.

This invention also relates to a syringe **300** as described above, said syringe **300** being preferably equipped with a clip **305** as described above, and said syringe **300** being equipped with no sheath **100** and no injection system according to the invention.

In one embodiment, the syringe **300** of the present invention comprises a cartridge **400** which is immobilized in radial direction via demolding clips **303** and the back of the syringe **304.**

In one embodiment, the syringe **300** of the present invention comprises a plunger **310** equipped with a plunger seal **312** comprising lips, wherein said lips may bend backwards for an easier introduction into the cartridge **400,** said plunger seal **312** being optionally overmolded on the plunger **310.**

In one embodiment, the syringe **300** of the present invention comprises soft overmoldings installed in the thumb area **311** and in the grip area **302** of said syringe **300.**

In one embodiment, the syringe **300** of the present invention comprises a precut **306** located substantially at the distal end of the syringe **300,** made of a series of perforations or one or more circumferential grooves, preferably one circumferential groove.

In another embodiment, the syringe **300** of the present invention comprises the following features:
- the cartridge **400** is immobilized in radial direction via demolding clips **303** and the back of the syringe **304;** and
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310.**

In one embodiment, the syringe **300** of the present invention comprises the following features:
- the cartridge 400 is immobilized in radial direction via demolding clips **303** and the back of the syringe **304;** and
- said syringe **300** comprises soft overmoldings installed in the thumb area **311** and in the grip area **302** of said syringe **300.**

In one embodiment, the syringe **300** of the present invention comprises the following features:
- the cartridge **400** is immobilized in radial direction via demolding clips **303** and the back of the syringe **304;** and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;** and
- said syringe **300** comprises soft overmoldings installed in the thumb area **311** and in the grip area **302** of said syringe **300.**

In one embodiment, the syringe **300** of the present invention comprises the following features:
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;** and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- said syringe **300** comprises soft overmoldings installed in the thumb area **311** and in the grip area **302** of said syringe **300**; and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- the cartridge **400** is immobilized in radial direction via demolding clips **303** and the back of the syringe **304;**
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;** and
- said syringe **300** comprises soft overmoldings installed in the thumb area **311** and in the grip area **302** of said syringe **300.**

In one embodiment, the syringe **300** of the present invention comprises the following features:
- the cartridge **400** is immobilized in radial direction via demolding clips **303** and the back of the syringe **304;**
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;** and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- the cartridge **400** is immobilized in radial direction via demolding clips **303** and the back of the syringe **304;**
- said syringe **300** comprises soft overmoldings installed in the thumb area 311 and in the grip area **302** of said syringe **300;** and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;**
- said syringe **300** comprises soft overmoldings installed in the thumb area **311** and in the grip area **302** of said syringe **300;** and
- said syringe **300** has a precut **306** located substantially at the distal end of the syringe **300,** made of a series of perforations or one or more circumferential grooves, preferably one circumferential groove.

In one embodiment, the syringe **300** of the present invention comprises the following features:
- the cartridge **400** is immobilized in radial direction via demolding clips **303** and the back of the syringe **304;**
- the plunger **310** of said syringe **300** is equipped with a plunger seal **312** comprising lips, wherein said lips may bend backwards for an easier introduction into the cartridge **400,** said plunger seal **312** being optionally overmolded on the plunger **310;**

- said syringe **300** comprises soft overmoldings installed in the thumb area **311** and in the grip area **302** of said syringe **300;** and
- said syringe **300** has a precut **306** located substantially at the distal end of the syringe **300,** made of a series of perforations or one or more circumferential grooves, preferably one circumferential groove.

This invention further relates to a manufacturing device, preferably a mould or an assembly machine, for manufacturing a sheath **100,** a hub **200,** and/or a syringe **300** according to the invention.

## Claims

1. Protective sheath (100) for a cannula (210) arranged on a hub (200) configured to be fitted onto a syringe (300), said sheath (100) comprising female means intended to cooperate with male means (201) of the hub (200) for guiding thereof, and **characterized in that**:
- said female means comprise a retracted position before use RBU, a retracted position during use RU, and a retracted position after use RAU, wherein the whole length of the cannula (210) is inside said sheath (100), said female means including primary locking means (120) for temporary locking said male means (201) in the RBU position, secondary locking means (140) for temporary locking said male means (201) in the RU position, and final locking means (170) for permanently locking said male means (201) in the RAU position;
- said female means comprise an ejection position E, wherein whole or part of the cannula (210) protrudes out of said sheath (100); and
- the RBU, RU and RAU positions are substantially transversally aligned.

2. Protective sheath (100) according to claim 1, wherein female means include:
- primary transition means (110) for insertion of male means (201) of the hub (200) within the sheath (100) and for guiding said male means (201) to the RBU position;
- primary locking means (120) for temporary locking said male means (201) in the RBU position;
- secondary transition means (130) for guiding said male means (201) from the RBU to the RU position;
- secondary locking means (140) for temporary locking said male means (201) in the RU position;
- guiding means (150), for guiding said male means (201) forth from the RU position to the E position and back from the E position to the RU position;
- tertiary transition means (160) for guiding said male means (201) from the RU position to the RAU position; and/or
- final locking means (170), for permanently locking said male means (201) in the RAU position.

3. Protective sheath (100) according to claim **1** or claim **2,** wherein primary transition means (110) are one-way transition means comprising anti-return means.

4. Protective sheath (100) according to any one of claims **1** to **3,** wherein secondary transition means (130) includes translation/rotation guiding means (131) for guiding male means (201) from the RBU position to a transitory position T, and longitudinal guiding means (132) for guiding said male means (201) from the T position to the RU position.

5. Protective sheath (100) according to claim **4,** wherein longitudinal guiding means (132) are one-way transition means comprising anti-return means.

6. Protective sheath (100) according to any one of claims **1** to **5,** wherein tertiary transition means (160) include rotational guiding means.

7. Protective sheath (100) according to any one of claims **1** to **6,** further comprising anti-slipping means, including:
- inclined planes (121, 133, 171, 172) and/or at least one rib (106); and/or
- at least one rib (107) for preventing dismounting of the hub (200) from said sheath (100).

8. Protective sheath (100) according to any one of claims **1** to **7,** further comprising gripping means (101) which are ribs covered by a grinding or an erosion grain such as VDI 20 to 30, located on the external surface (105) of said sheath (100).

9. Injection system comprising the protective sheath (100) according to any one of claims **1** to **8,** and a hub (200) inserted within said sheath (100).

10. Injection system according to claim **9,** wherein the hub (200) comprises:
- male means (201), adapted to cooperate with female means of a sheath (100) wherein the hub (200) is configured to be inserted; and
- optionally a cannula (210), protruding at both ends of the hub (200).

11. Syringe (300) including a protective sheath (100) according to any one of claims **1** to **8** or an injection system according to claim **9** or claim **10.**

12. Syringe (300) according to claim **11,** which is configured to hold a cartridge (400) and wherein the cartridge (400) is immobilized in axial direction at the neck (402) of said cartridge (400), via a flexible clip (305).

13. Syringe (300) according to claim **11** or claim **12,** which is a disposable syringe having a precut (306) located substantially at the distal end of the syringe (300), made of a series of perforations or one or more circumferential grooves.

14. Syringe (300) according to any one of claims **11** to **13,** wherein the cartridge (400) is immobilized in radial direction and wherein the plunger (310) of said syringe (300) is equipped with a plunger seal (312) comprising lips (313).

## Patentansprüche

1. Schutzhülle (100) für eine an einem Ansatzstück (200) angeordnete Kanüle (210), konfiguriert, um auf eine Spritze (300) aufgesetzt zu werden, wobei die Hülle (100) Aufnahmemittel umfasst, die dazu bestimmt sind, mit Einsteckmitteln (201) des Ansatzstücks (200) zum Führen davon zusammenzuwirken, und **dadurch gekennzeichnet, dass**:
- die Aufnahmemittel eine zurückgezogene Position vor Verwendung RBU, eine zurückgezogene Position während Verwendung RU und eine zurückgezogene Position nach Verwendung RAU umfassen, wobei die ganze Länge der Kanüle (210) im Inneren der Hülle (100) vorliegt, die Aufnahmemittel primäre Verriegelungsmittel (120) zum vorübergehenden Verriegeln der Einsteckmittel (201) in der RBU-Position, sekundäre Verriegelungsmittel (140) zum vorübergehenden Verriegeln der Einsteckmittel (201) in der RU-Position und endgültige Verriegelungsmittel (170) zum permanenten Verriegeln der Einsteckmittel (201) in der RAU-Position einschließen;
- die Aufnahmemittel eine Auswurfposition E umfassen, wobei die Kanüle (210) ganz oder teilweise aus der Hülle (100) herausragt; und
- die RBU-, RU- und RAU-Positionen im Wesentlichen querverlaufend ausgerichtet sind.

2. Schutzhülle (100) nach Anspruch **1,** wobei Aufnahmemittel einschließen:
- primäre Übergangsmittel (110) zum Einsetzen von Einsteckmitteln (201) des Ansatzstücks (200) innerhalb der Hülle (100) und zum Führen der Einsteckmittel (201) zu der RBU-Position;
- primäre Verriegelungsmittel (120) zum vorübergehenden Verriegeln der Einsteckmittel (201) in der RBU-Position;
- sekundäre Übergangsmittel (130) zum Führen der Einsteckmittel (201) aus der RBU- zu der RU-Position;
- sekundäre Verriegelungsmittel (140) zum vorübergehenden Verriegeln der Einsteckmittel (201) in der RU-Position;
- Führungsmittel (150) zum Führen der Einsteckmittel (201) vorwärts aus der RU-Position in die E-Position und zurück aus der E-Position in die RU-Position;
- tertiäre Übergangsmittel (160) zum Führen der Einsteckmittel (201) aus der RU-Position zu der RAU-Position; und/oder
- endgültige Verriegelungsmittel (170) zum permanenten Verriegeln der Einsteckmittel (201) in der RAU-Position.

3. Schutzhülle (100) nach Anspruch 1 oder Anspruch 2, wobei primäre Übergangsmittel (110) Einweg-Übergangsmittel sind, die Anti-Rückkehr-Mittel umfassen.

4. Schutzhülle (100) nach einem der Ansprüche **1** bis **3,** wobei sekundäres Übergangsmittel (130) Translations-/Rotationsführungsmittel (131) zum Führen von Einsteckmitteln (201) aus der RBU-Position in eine transitorische Position T und längsgerichtete Führungsmittel (132) zum Führen der Einsteckmittel (201) aus der T-Position in die RU-Position einschließt.

5. Schutzhülle (100) nach Anspruch **4,** wobei längsgerichtete Führungsmittel (132) Einweg-Übergangsmittel sind, die Anti-Rückkehr-Mittel umfassen.

6. Schutzhülle (100) nach einem der Ansprüche **1** bis **5,** wobei tertiäre Übergangsmittel (160) rotatorische Führungsmittel umfassen.

7. Schutzhülle (100) nach einem der Ansprüche **1** bis **6,** weiter umfassend Anti-Abrutsch-Mittel, einschließlich:
- geneigte Ebenen (121, 133, 171, 172) und/oder mindestens eine Rippe (106); und/oder
- mindestens eine Rippe (107) zum Verhindern von Demontage des Ansatzstücks (200) von der Hülle (100).

8. Schutzhülle (100) nach einem der Ansprüche **1** bis **7,** weiter umfassend Greifmittel (101), die durch ein Schleif- oder ein Erosionskorn wie VDI 20 bis 30 bedeckte Rippen sind, die sich auf der externen Oberfläche (105) der Hülle (100) befinden.

9. Injektionssystem, umfassend die Schutzhülle (100) nach einem der Ansprüche **1** bis **8** und ein innerhalb der Hülle (100) eingesetztes Ansatzstück (200).

10. Injektionssystem nach Anspruch **9,** wobei das Ansatzstück (200) umfasst:
- Einsteckmittel (201), angepasst, um mit Aufnahmemitteln einer Hülle (100) zusammenzuwirken, wobei das Ansatzstück (200) konfiguriert ist, um eingesetzt zu werden; und
- wahlweise eine an beiden Enden des Ansatzstücks (200) herausragende Kanüle (210).

11. Spritze (300), die eine Schutzhülle (100) nach einem der Ansprüche **1** bis **8** oder ein Injektionssystem nach Anspruch **9** oder Anspruch **10** einschließt.

12. Spritze (300) nach Anspruch **11,** die konfiguriert ist, um eine Kartusche (400) zu halten und wobei die Kartusche (400) in axialer Richtung an dem Hals (402) der Kartusche (400) mittels einer flexiblen Klammer (305) immobilisiert ist.

13. Spritze (300) nach Anspruch **11** oder Anspruch **12,** die eine Einwegspritze mit einer Vortrennlinie (306), die sich im Wesentlichen an dem distalen Ende der Spritze (300) befindet, hergestellt aus einer Reihe von Perforationen oder einer oder mehreren umlaufenden Rillen, ist.

14. Spritze (300) nach einem der Ansprüche **11** bis **13,** wobei die Kartusche (400) in einer Radialrichtung immobilisiert ist und wobei der Kolben (310) der Spritze (300) mit einer Kolbendichtung (312), die Lippen (313) umfasst, ausgestattet ist.

## Revendications

1. Gaine de protection (100) pour une canule (210) disposée sur un embout (200) configuré pour être emboîté sur une seringue (300), ladite gaine (100) comportant des moyens femelles destinés à coopérer avec des moyens mâles (201) de l'embout (200) pour son guidage, et **caractérisée en ce que** :
- lesdits moyens femelles comprennent une position rétractée avant utilisation RBU, une position rétractée pendant utilisation RU et une position rétractée après utilisation RAU, dans lesquelles toute la longueur de la canule (210) est à l'intérieur de ladite gaine (100), lesdits moyens femelles comprenant des moyens de verrouillage primaires (120) pour verrouiller temporairement lesdits moyens mâles (201) dans la position RBU, des moyens de verrouillage secondaires (140) pour verrouiller temporairement lesdits moyens mâles (201) dans la position RU, et des moyens de verrouillage finaux (170) pour verrouiller de manière permanente lesdits moyens mâles (201) dans la position RAU ;
- lesdits moyens femelles comprennent une position d'éjection E, dans laquelle tout ou partie de la canule (210) fait saillie hors de ladite gaine (100); et
- les positions RBU, RU et RAU sont sensiblement alignées transversalement.

2. Gaine de protection (100) selon la revendication **1,** dans laquelle les moyens femelles comprennent :
- des moyens de transition primaires (110) pour l'insertion des moyens mâles (201) de l'embout (200) dans la gaine (100) et pour le guidage desdits moyens mâles (201) vers la position RBU ;
- des moyens de verrouillage primaires (120) pour temporairement verrouiller lesdits moyens mâles (201) dans la position RBU;
- des moyens de transition secondaires (130) pour le guidage desdits moyens mâles (201) de la position RBU à la position RU ;
- des moyens de verrouillage secondaires (140) pour temporairement verrouiller lesdits moyens mâles (201) dans la position RU;
- des moyens de guidage (150) pour le guidage desdits moyens mâles (201) en avant de la position RU à la position E et en arrière de la position E à la position RU ;
- des moyens de transition tertiaires (160) pour le guidage desdits moyens mâles (201) de la position RU à la position RAU ; et/ou
- des moyens de verrouillage finaux (170) pour verrouiller de manière permanente lesdits moyens mâles (201) dans la position RAU.

3. Gaine de protection (100) selon la revendication **1** ou la revendication **2,** dans laquelle les moyens de transition primaires (110) sont des moyens de transition à sens unique comprenant des moyens anti-retours.

4. Gaine de protection (100) selon l'une quelconque des revendications **1** à **3,** dans laquelle les moyens de transition secondaires (130) incluent des moyens de guidage en translation/rotation (131) pour guider les moyens mâles (201) de la position RBU à une position transitoire T, et des moyens de guidage longitudinal (132) pour guider lesdits moyens mâles (201) de la position T à la position RU.

5. Gaine de protection (100) selon la revendication **4,** dans laquelle les moyens de guidage longitudinal (132) sont des moyens de transition à sens unique comprenant des moyens anti-retours.

6. Gaine de protection (100) selon l'une quelconque des revendications **1** à **5,** dans laquelle les moyens de transition tertiaires (160) incluent des moyens de guidage rotationnel.

7. Gaine de protection (100) selon l'une quelconque des revendications **1** à **6,** comprenant en outre des moyens antidérapants, incluant :
- des plans inclinés (121, 133, 171, 172) et/ou au moins une nervure (106) ; et/ou
- au moins une nervure (107) pour empêcher le démontage de l'embout (200) de ladite gaine (100).

8. Gaine de protection (100) selon l'une quelconque des revendications **1** à **7,** comprenant en outre des moyens de préhension (101) qui sont des nervures recouvertes d'un grain de meulage ou d'érosion tel que VDI 20 à 30 ; situées sur la surface externe (105) de ladite gaine (100).

9. Système d'injection comprenant la gaine de protection (100) selon l'une quelconque des revendications **1** à **8,** et un embout (200) inséré dans ladite gaine (100).

10. Système d'injection selon la revendication **9,** dans lequel l'embout (200) comprend :
- des moyens mâles (201) configurés pour coopérer avec des moyens femelles d'une gaine (100) dans laquelle l'embout (200) est configuré pour être inséré ; et
- optionnellement, une canule (210) faisant saillie aux deux extrémités de l'embout (200).

11. Seringue (300) incluant une gaine de protection (100) selon l'une quelconque des revendications **1** à **8** ou un système d'injection selon la revendication **9** ou la revendication **10.**

12. Seringue (300) selon la revendication **11,** qui est configurée pour maintenir une cartouche (400) et dans laquelle la cartouche (400) est immobilisée selon une direction axiale au niveau du col (402) de ladite cartouche (400), via un clip flexible (305).

13. Seringue (300) selon la revendication **11** ou la revendication **12,** qui est une seringue jetable ayant une prédécoupe (306) située sensiblement à l'extrémité distale de la seringue (300), constituée d'une série de perforations ou d'une ou plusieurs rainures circonférentielles.

14. Seringue (300) selon l'une quelconque des revendications **11** à **13,** dans laquelle la cartouche (400) est immobilisée dans le sens radial et dans laquelle le piston (310) de ladite seringue (300) est équipé d'un joint de piston (312) comprenant des lèvres (313).
